# FASCICULE DE BREVET EUROPEEN

(11) **EP 1 089 703 B1**
(45) Date de publication et mention de la délivrance du brevet: **22.05.2002**
(21) Numéro de dépôt: 99957144.1
(22) Date de dépôt: 21.06.1999
(51) Int. Cl.: A61K 7/035, C09C 3/12

(54) **PRODUIT SOLIDE MANIPULABLE A BASE DE POUDRE MINERALE HYDROPHOBE**
HANDHABBARES FESTES PRODUKT AUF BASIS VON HYDROPHOBEM MINERALPULVER
SOLID PRODUCT CAPABLE OF BEING HANDLED BASED ON HYDROPHOBIC MINERAL POWDER

(30) Priorité: 23.06.1998 FR 9807936; 01.10.1998 FR 9812307
(43) Date de publication de la demande: 11.04.2001
(73) Titulaire: TALC DE LUZENAC, 09250 Luzenac (FR)
(72) Inventeur: ARSEGUEL, Didier, F-31450 Deyme (FR); GOFFINET, Pierre, Charles, F-91190 Gif sur Yvette (FR); RIVIERE, Brigitte, F-86000 Poitiers (FR)
(74) Mandataire: Cabinet BARRE LAFORGUE & associés
(86) Numéro de dépôt international: FR9901481
(87) Numéro de publication internationale: WO9966885

(56) Documents cités:
- EP-A- 0 587 415
- WO-A-96/30448
- US-A- 4 804 538
- US-A- 4 837 011
- US-A- 5 023 075

## Description

L'invention concerne un produit solide à base de poudre minérale hydrophobe, en particulier talc ou mica, ayant une forme propre prédéterminée se prêtant aux manipulations, notamment en forme de stick ou bâtonnet. Elle vise en particulier un produit solide à usage cosmétologique ou hygiénique, permettant de réaliser un dépôt de talc ou de mica sur la peau par contact et frottement doux. L'invention s'étend à un procédé de fabrication permettant d'obtenir un tel produit avec une rigidité et une cohésion compatibles avec une utilisation sans risque de rupture où d'effritement.

Le talc est fréquemment utilisé sous forme de poudre comme produit actif en cosmétologie en raison de ses propriétés émollientes. Le dépôt de talc ou le film fin formé protège la peau contre les rougeurs et irritations, protège l'épidémie des frottements et absorbe les excès de transpiration. Cette utilisation très ancienne s'effectue par une pulvérisation de poudre sur la surface à traiter et s'avère peu commode en pratique : la pulvérisation engendre un nuage de poudre plus ou moins important selon la finesse du talc, qui pollue l'atmosphère, souille d'autres surfaces que la surface à traiter et conduit à des pertes de matières.

Par ailleurs, de la poudre de talc ou de mica est incorporée comme charge dans de nombreuses formulations cosmétologiques. Cette poudre minérale est généralement pressée avec des produits actifs et des liants gras du type paraffines, lanolines, glycérines, stéarates, pour obtenir une pâte friable qui est disposée dans un godet ou une barquette en vue de son utilisation généralement au moyen d'un pinceau (en particulier en ce qui concerne les produits de maquillage). Dans ces produits, le talc ou le mica constitue une charge qui sert essentiellement à diluer le ou les produits actifs auxquels il est incorporé. Cette pâte contenue dans le godet ou la barquette n'a aucune tenue propre, mais compte tenu de son conditionnement, une faible cohésion de même qu'une friabilité élevée sont admissibles, ce qui permet de prévoir des proportions de talc ou de mica de l'ordre de 40 à 50 % pouvant exceptionnellement atteindre 80 % dans certains produits (WO 97/04737 visant une composition "à hauts niveaux de talc" ; "Handbook of Cosmetic Science and Technology, 1^{st} edition, 1993, p. 134-145, US 4 837 011, EP 0 587 415, WO 98.30195). Une proportion élevée de talc ou mica combinée à une proportion relativement réduite de liant gras limitent, lors de l'application, l'effet "gras" engendré par le produit.

Il existe, par ailleurs, des sticks pour lèvres (rouges à lèvres ou sticks d'enduction) qui comprennent des produits actifs et des liants gras et permettent de déposer sur les lèvres une couche grasse d'apprêt ou de protection.

Ces sticks ont une tenue suffisante pour permettre de les manipuler, mais ne comportent généralement pas de talc ou de mica (lesquels sont connus pour avoir des effets défavorables sur la cohésion des particules). L'ouvrage "Handbook of Cosmetic Science and Technology" cité plus haut fournit les formulations les plus courantes de ce type de sticks (p. 147-155). Il est à noter toutefois que la revue "Cosmetics and Toiletrics, April 1992", donne, p. 95 et 96, une formulation de stick pour lèvres contenant un petit pourcentage de mica (5 %) ou de talc (3,65 %).

Les poudres minérales de talc ou de mica, et plus généralement les poudres minérales hydrophobes, sont donc connues pour s'agglomérer difficilement et donner des gâteaux très friables dès que la proportion de poudre minérale devient élevée dans le produit : ceci explique que, depuis plus d'un siècle, le talc est utilisé sous forme de poudre lorsqu'il est destiné à être appliqué seul (ou en proportion très majoritaire) comme produit actif cosmétologique ou hygiénique. Depuis quelques dizaines d'années, le développement des nombreuses formulations précédemment visées, comprenant du talc ou mica comme charge, n'ont fait que confirmer cet enseignement, puisque les produits à forte charge de talc ou mica sont des produits sans cohésion propre, nécessairement contenus dans des godets, barquettes ou autres conteneurs en vue de les appliquer au moyen d'un instrument tel que pinceau, et que les très rares produits en stick contenant du talc en renferment très peu.

Il est à noter que certains documents proposent de revêtir les particules de poudre hydrophobe pour en modifier les propriétés de surface (US 5 023 075, WO 96.30448) ; il ne semble pas toutefois que l'objectif de ce traitement soit la fabrication de produits solides ; en tout cas, de telles techniques ne constituent pas une solution au problème de l'invention, puisque les caractéristiques de la poudre naturelle initiale sont modifiées.

La présente invention se propose de fournir un produit solide à base de poudre minérale hydrophobe, telle que talc ou mica, qui se présente avec une forme rigide permettant de le manipuler en vue d'une application directe de composé minéral par contact avec la surface à traiter : l'invention vise ainsi à supprimer les inconvénients liés aux traitements précédemment évoqués par pulvérisation de poudre.

L'objectif de l'invention est en particulier de fournir un produit en stick ou autre forme manipulable, qui soit essentiellement constitué à partir d'une poudre minérale à base de talc ou de mica (c'est-à-dire dans lequel les autres composants représentent moins de 20 % en poids du produit) et qui bénéficie des caractères suivants :
. cohésion et tenue propre compatibles avec un usage par contact sans rupture ni effritement du produit solide,
. bonne distribution de talc ou de mica par contact avec la surface à traiter,
. impression sèche lors de l'application sans effet gras.

Un autre objectif de l'invention est d'éviter l'effet dit "de cirage", qui consiste en une diminution de la distribution après plusieurs utilisations.

A cet effet, le procédé conforme à l'invention pour fabriquer un tel produit solide de forme propre et de cohésion le rendant manipulable, se caractérise en ce qu'on imprègne la poudré minérale hydrophobe, en particulier poudre minérale à base de talc ou mica, au moyen d'un liant liquide volatil dans des proportions pondérales telles que la proportion de liant liquide volatil soit au moins égale à 5 % et la proportion de poudre minérale au moins égale à 80 %, et en ce qu'on presse la poudre imprégnée obtenue de façon à la mettre en forme et à lui conférer une cohésion assurant la rigidité du produit.

Par "poudre minérale à base de talc ou de mica", on entend une poudre dans laquelle le talc ou le mica ou un mélange des deux représente au moins 75 % en poids de la poudre (généralement plus de 85 %, préférentiellement plus de 95 %, voire 100 %), les autres composés éventuels étant des composés minéraux tels que kaolin, carbonate, ... ou éventuellement organique tel que amidon, .... Par "talc", on entend soit le minéral silicate de magnésium hydraté, soit le minéral chlorite (silicate de magnésium et d'aluminium hydraté), soit un mélange des deux. Par "mica", on entend les micas alumineux tels que séricite, muscovite, les micas magnésiens tels que phlogopite, ainsi que les illites alumineuses ou magnésiennes dérivant des minéraux précédents par des substitutions variables de Aℓ à Si d'une part, de Fe à Aℓ ou Mg d'autre part.

Par "liant liquide volatil", on entend un liant qui est liquide à température et à pression ordinaires et dont la volatilité définie par la norme DIN 53249 est telle que la perte de poids soit supérieure ou égale à 30 % après 100 minutes à 25 °C. Ce type de liant volatil présente une viscosité toujours inférieure à 50 mPa.s (50 centipoises).

Par le terme "presser", on entend toute opération de compression des grains de poudre, en particulier opération de pressage dans tout système de presse, opération d'extrusion, ..., le produit solide obtenu étant ensuite extrait de la presse pour être conditionné selon sa forme et l'application envisagée.

Les essais ont démontré qu'il était possible de réaliser, par le procédé de l'invention, des produits à base de poudre minérale hydrophobe telle que poudre de talc ou de mica, qui présentent une rigidité et une cohésion compatibles avec une bonne stabilité de forme et une manipulation sans rupture ni effritement (à la fabrication lors du démoulage et à l'usage), et ce en apportant une impression sèche lorsqu'ils sont appliqués sur la peau (absence d'effet "gras") : le talc peut ainsi être utilisé par contact pour agir comme produit actif dans les mêmes conditions que les produits en poudre de talc pulvérisables traditionnels. Lors du contact avec la surface à traiter, le produit se délite en surface conduisant à une bonne distribution du composé minéral et à un dépôt ou film propre à assurer le traitement souhaité. Un effet de cirage n'est pas observé même au terme d'un grand nombre d'utilisations (sauf si les pressions de pressage sont très élevées).

Les pressions optimales de pressage de la poudre imprégnée sont fonction de la poudre minérale et du liant liquide volatil utilisé et de sa proportion (ainsi que des additifs éventuellement ajoutés) ; une pression comprise entre 200 et 700 kg/cm² donne généralement de bons résultats pour des poudres de talc et de mica. La poudre imprégnée est disposée dans un moule, de forme conjuguée à la forme désirée, et la pression précitée est en particulier appliquée au moyen d'un piston (avec évacuation de l'air).

La forme donnée au produit lors du pressage est préférentiellement celle d'un stick ou bâtonnet mais peut être différente selon l'application : pain, cylindre...

Le liant liquide volatil utilisé est avantageusement un silicone cyclique liquide, en particulier de la famille des cyclométhicones à faible degré de polymérisation tel que sa viscosité soit inférieure à 10 mPa.s (10 centipoises) et préférentiellement inférieure ou égale à 5 mPa.s (5 centipoises). Ces composés conduisent à un produit d'excellente qualité (tenue propre/distribution/ impression sèche/absence d'effet de cirage).

Il est possible d'ajouter au produit un faible pourcentage de liant gras organique sous forme de poudre, en particulier poudre d'un stéarate métallique. Cette adjonction augmente la qualité de distribution du produit par contact, mais augmente également l'effet "gras" et la friabilité. On a pu observer qu'une proportion pondérale de liant gras inférieure à la proportion de liant liquide volatil conduisait à de bons résultats ; un excellent compromis consiste à ajouter à la poudre minérale hydrophobe sensiblement entre 6 % et 9 % en poids de liant liquide volatil et entre 2 % et 3 % en poids de poudre de stéarate métallique. Il est à noter que les produits formés avec ces proportions de liant gras mais en l'absence de liant liquide volatil (ou avec une proportion réduite inférieure à celle du liant gras) présentent une friabilité très marquée incompatible avec l'obtention d'une forme stable manipulable (en cas d'absence totale de liant liquide volatil, un grand nombre de produits casse au démoulage).

D'autres additifs tels que parfum, émollient, peuvent également être ajoutés à la poudre en faible proportion (c'est-à-dire inférieure ou égale à 2 %).

L'invention s'étend au produit nouveau constitué par une forme solide manipulable, en particulier stick, propre à engendrer un dépôt de composé minéral tel que talc ou mica par contact, ledit produit comprenant une poudre minérale hydrophobe pressée et imprégnée par un liant liquide volatil, la poudre minérale représentant entre 80 % et 95 % du poids total et le liant liquide volatil au moins 5 % dudit poids. La poudre minérale est en particulier une poudre à base de talc ou de mica en raison des propriétés cosmétologiques ou hygiéniques de ces composés. Dans le cas du talc, il est ainsi possible de réaliser un stick contenant, en matière solide, au moins 95 % de talc, notamment 100 % (poids de talc rapporté au poids de matières solides du produit).

Les exemples qui suivent illustrent la mise en oeuvre du procédé de l'invention et les caractéristiques des produits obtenus. Tous les exemples et les essais comparatifs ont été mis en oeuvre au moyen d'un dispositif de pressage tel que représenté sur le dessin ; sur ce dessin :
- la figure 1 est en coupe schématique dudit dispositif de pressage,
- la figure 2 schématise le stick réalisé,
- la figure 3 schématise en coupe le stick dans un conditionnement facilitant son usage.

Le dispositif de pressage comprend un cylindre 1 possédant une chambre de compression faisant office de moule, dans laquelle est susceptible de pénétrer un piston 2 lié à un plateau supérieur de presse 3. Le cylindre 1 repose sur un support amovible 4 qui l'obture en partie basse et est pourvu d'un fritté 5 et un conduit 6 d'évacuation d'air. Après passage, le produit obtenu est extrait du moule et conditionné au moyen d'un support ou conditionnement adapté à sa forme, permettant sa préhension.

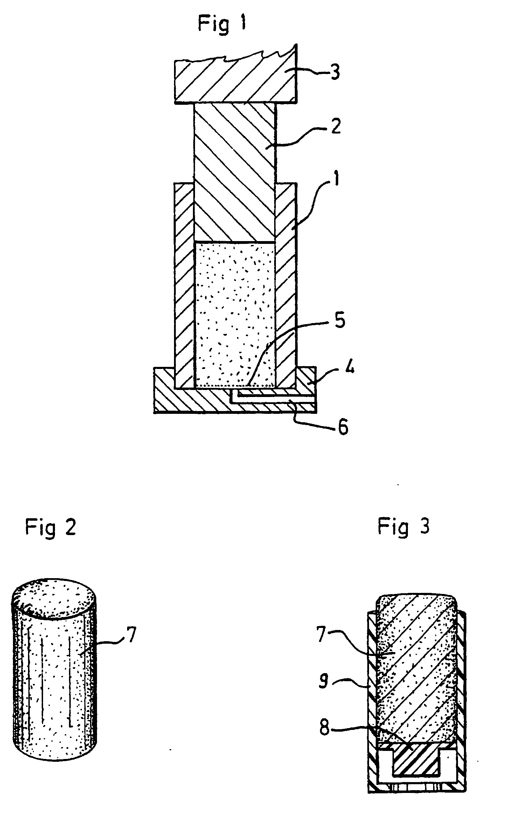

Les sticks fabriqués dans les exemples sont de forme cylindrique tel que le stick 7 schématisé à la figure 2, de diamètre égal à 37 mm et de hauteur égale à 50 mm.

### EXEMPLE 1

Dans cet exemple, on utilise un talc ayant une répartition granulométrique caractérisée par un diamètre D₅₀ de 15 microns, un diamètre D₉₅ de 40 microns et un diamètre de coupure D₉₉ de 75 microns (par "diamètre moyen D₅₀ ou diamètre D₉₅", on entend un diamètre de particules tel que respectivement 50 % ou 95 % des particules soient de taille inférieure à cette valeur ; par "diamètre de coupure D₉₉", on entend un diamètre de particule tel que moins de 1 % des particules ont une taille supérieure à cette valeur.

Ce talc contient 94,5 % de silicate de magnésium hydraté, 3 % de chlorite (silicate de magnésium et d'aluminium hydraté) et 1,5 % de carbonate de calcium. La masse volumique apparente non tassée de la poudre est 0,43 g/cm³.

Cette poudre de talc est mélangée avec une poudre de stéarate de magnésium de granulométrie analogue à celle du talc. L'ensemble est progressivement imprégné au moyen de cyclométhicone pentamère qui est un composé cyclique volatil liquide à température et pression ambiantes (volatilité de 84 % selon la définition donnée à la page 3). La poudre imprégnée est ensuite homogénéisée.

La poudre imprégnée obtenue présente la composition pondérale suivante : 90 % de talc, 7,5 % de liant liquide volatil et 2,5 % de stéarate.

Une masse de 115 g de poudre imprégnée est disposée dans le cylindre 1 et on applique une pression de 500 kg/cm² au moyen du piston 2. L'ensemble est maintenu sous presse 15 mn environ.

Le produit obtenu est démoulé en le fond amovible 4. On obtient un stick de forme cylindrique tel que représenté à la figure 2 qui a une bonne tenue et une cohésion propre, sans tendance à s'effriter, ou se casser.

Ce stick peut être disposé dans un conditionnement classique 9, comprenant un poussoir 8 pour permettre de faire sortir l'extrémité du stick au fur et à mesure de son usure (figure 3).

Ce stick est soumis à des tests qualitatifs d'évaluation des paramètres suivants : friabilité, distribution du talc, cirage et toucher.

Le cirage est évalué par la distribution obtenue après 10 usages. Plusieurs appréciations sont données :
F = friabilité : très friable, moyenne, légère, nulle
D = distribution : très bonne, bonne, moyenne, nulle
C = cirage : marqué, léger, très léger, nul
T = toucher : sec, léger gras, gras, très gras

On peut estimer qu'un produit d'hygiène en stick à base de talc est satisfaisant si sa friabilité est légère ou nulle, sa distribution très bonne, bonne ou moyenne, son cirage nul ou très léger, et son toucher sec (à la rigueur léger gras). Le tableau suivant donne les caractéristiques du produit fabriqué dans cet exemple.

| | | | |
|---|---|---|---|
| F | D | C | T |
| nulle | bonne | nul | sec |

Ce produit est satisfaisant. Il ne s'effrite pas, ni ne se casse. Il donne une distribution de talc correcte même après plusieurs usages, et son toucher est équivalent à celui d'une pulvérisation de poudre de talc.

### EXEMPLE 2

Cet exemple reproduit la mise en oeuvre de l'exemple 1 avec une composition différente de la poudre imprégnée :
- 90 % de talc
- 10 % de cyclométhicone pentamère

Ces résultats obtenus sont les suivants :

| | | | |
|---|---|---|---|
| F | D | C | T |
| nulle | moyenne | nul | sec |

Ce produit convient également. La distribution est légèrement moins bonne.

### EXEMPLE 3

Mêmes conditions de mise en oeuvre mais avec une proportion de liant volatil liquide moindre :
- 95 % de talc
- 5 % de cyclométhicone pentamère

Les résultats obtenus sont les suivants :

| | | | |
|---|---|---|---|
| F | D | C | T |
| légère | bonne | nul | sec |

Ce produit demeure satisfaisant mais sa friabilité est supérieure à celle de ceux des exemples 1 et 2 et une proportion moindre de liant liquide volatil entraînerait des risques d'effritement ou de cassure (comme le montrent les essais comparatifs en l'absence de liant).

### EXEMPLE 4

Cet exemple est mis en oeuvre dans les mêmes conditions que précédemment, avec une poudre imprégnée, de composition:
- 90 % de talc
- 7,5 % de cyclométhicone tétramère (volatilité 100 % selon la définition précitée)
- 2,5 % de stéarate de magnésium.

Les résultats obtenus sont les suivants :

| | | | |
|---|---|---|---|
| F | D | C | T |
| légère | moyenne | nul | sec |

Ce produit est satisfaisant.

### EXEMPLE 5

Un produit est fabriqué de façon analogue avec la formulation suivante :
- 89,8 % de talc
- 7,5 % de cyclométhicone pentamère
- 2,5 % de stéarate de magnésium
- 0,12 % de parfum (réf. "PE8034")
- 0,025 % d'éthanol

Le parfum référence "PE 8034" ("Douceur coton" de la Société "QUEST") est solubilisé dans l'éthanol et est ajouté après le liant volatil liquide.

Les résultats sont les mêmes qu'à l'exemple 1.

### EXEMPLE 6

Dans ce produit, on augmente la proportion de liant volatil et celle du stéarate de magnésium.
- 85 % de talc
- 7,5 % de cyclométhicone tétramère
- 7,5 % de stéarate de magnésium.

Les résultats obtenus sont les suivants :

| | | | |
|---|---|---|---|
| F | D | C | T |
| légère | moyenne | nul | léger gras |

Ce produit donne un toucher avec une légère impression de gras, qui peut le faire exclure dans certaines applications (notamment si l'on recherche une impression identique à celle d'une poudre de talc pulvérisée).

### Essai comparatif (talc seul)

Dans cet essai, on met en oeuvre le procédé de pressage en utilisant une poudre constituée uniquement de talc, et on la presse, d'une part, à 500 kg/cm² comme précédemment, d'autre part, à 1 000 kg/cm².

Dans les deux cas, la poudre s'effrite immédiatement au démoulage sans possibilité de former un stick.

### Essai comparatif (talc et stéarate)

Dans cet essai, on met en oeuvre le procédé avec un mélange de poudre, de composition suivante :
- 90 % de talc
- 10 % de stéarate de magnésium

Le stick se casse au démoulage et un morceau de stick subit les tests d'évaluation :

| | | | |
|---|---|---|---|
| F | D | C | T |
| très friable | très bonne | nul | gras |

Ce produit n'est pas utilisable en stick, d'une part, pour des questions de tenue et de cohésion (fabrication et usage), d'autre part, pour son toucher trop gras (impression éloignée du toucher sec d'une poudre pulvérisée de talc).

### Essai comparatif (talc, stéarate, faible proportion de liant volatil)

Dans cet essai, on met en oeuvre le procédé avec une composition suivante :
- 90 % de talc
- 7,5 % de stéarate de magnésium
- 2,5 % de cyclométhicone tétramère

Le produit se démoule de façon satisfaisante sans cassure. Les résultats des tests sont les suivants :

| | | | |
|---|---|---|---|
| F | D | C | T |
| très friable | bonne | nul | léger gras |

Ce produit est beaucoup trop friable pour permettre une utilisation en stick.

### EXEMPLE 7

Dans cet exemple, on utilise un mica du type séricite ayant une répartition granulométrique caractérisée par un diamètre D₅₀ de 3,3 microns et un diamètre de coupure D₉₉ de 15 microns.

Cette poudre de mica est mélangée avec une poudre de stéarate de magnésium. L'ensemble est progressivement imprégné au moyen de cyclométhicone pentamère (volatilité de 84 % selon la définition donnée à la page 3). La poudre imprégnée est ensuite homogénéisée.

La poudre imprégnée obtenue présente la composition pondérale suivante : 85 % de mica, 11 % de liant liquide volatil et 4 % de stéarate.

Une masse de 135 g de poudre imprégnée est disposée dans le cylindre 1 et on applique une pression de 500 kg/cm² au moyen du piston 2. L'ensemble est maintenu sous presse 15 mn environ.

Le produit obtenu est démoulé en retirant le fond amovible 4. On obtient un stick de forme cylindrique qui a une bonne tenue et une cohésion propre, sans tendance à s'effriter, ou se casser.

Ce stick est soumis à des tests qualitatifs d'évaluation des paramètres suivants : friabilité, distribution du mica, cirage et toucher. L'appréciation s'effectue de façon identique à l'exemple 1.

Le tableau suivant donne les caractéristiques du produit fabriqué dans cet exemple.

| | | | |
|---|---|---|---|
| F | D | C | T |
| nulle | bonne | nul | sec |

Ce produit est satisfaisant. Il ne s'effrite pas, ni ne se casse. Il donne une distribution de mica correcte même après plusieurs usages, et son toucher est équivalent à celui d'un dépôt au pinceau.

### Essai comparatif (mica seul)

Dans cet essai, on met en oeuvre le procédé de pressage en utilisant une poudre constituée uniquement de mica du type séricite, identique à celle utilisée à l'exemple 7, et on la presse à 500 kg/cm².

La poudre est extrêmement friable et s'effrite partiellement au démoulage ; le stick imparfait formé perd sa cohésion à l'utilisation et ne possède pas la stabilité nécessaire pour une utilisation normale à plusieurs reprises.

## Revendications

1. Procédé de fabrication d'un produit solide de forme manipulable à base de composés minéraux hydrophobes, en particulier à usage cosmétologique ou hygiénique, **caractérisé en ce qu'**on imprègne une poudre contenant au moins 75% de composés minéraux hydrophobes au moyen d'un liant liquide volatil dans des proportions pondérales telles que la proportion de liant liquide volatil soit au moins égale à 5 % et la proportion de poudre au moins égale à 80 %, et **en ce qu'**on presse la poudre imprégnée obtenue de façon à la mettre en forme et à lui conférer une cohésion assurant la rigidité du produit.

2. Procédé selon la revendication 1, dans lequel la poudre est à base de talc ou de mica.

3. Procédé selon l'une des revendications 1 ou 2, **caractérisé en ce qu'**on utilise comme liant volatil un silicone cyclique liquide, de viscosité inférieure ou égale à 10 mPa.s (10 centipoises).

4. Procédé selon la revendication 3, **caractérisé en ce qu'**on utilise comme liant liquide volatil un silicone cyclique de la famille des cyclométhicones.

5. Procédé selon l'une des revendications 1, 2, 3 ou 4, **caractérisé en ce qu'**on presse la poudre imprégnée en la soumettant à une pression de pressage sensiblement comprise entre 200 et 700 kg/cm² dans un moule de forme conjuguée à la forme désirée, le produit solide obtenu étant extrait du moule et conditionné au moyen d'un support adapté à sa forme, permettant sa préhension.

6. Procédé selon l'une des revendications 1, 2, 3, 4 ou 5, **caractérisé en ce qu'**on ajoute à la poudre une poudre d'un liant gras organique dans une proportion pondérale inférieure à la proportion de liant liquide volatil.

7. Procédé selon la revendication 6, dans lequel on ajoute à la poudre un stéarate métallique.

8. Procédé selon la revendication 7, **caractérisé en ce qu'**on réalise la poudre imprégnée en ajoutant à la poudre sensiblement entre 6 % et 9 % en poids de liant liquide volatil et entre 2 % et 3 % en poids de poudre de stéarate métallique.

9. Procédé selon l'une des revendications précédentes, dans lequel on incorpore à la poudre un additif, en particulier parfum ou émollient, dans une proportion pondérale inférieure ou égale à 2 %.

10. Procédé selon l'une des revendications précédentes, dans lequel la poudre contient en poids au moins 95 % de talc.

11. Produit solide ayant une forme propre et une cohésion le rendant manipulable, en particulier en forme de stick, propre à engendrer un dépôt minéral par contact, **caractérisé en ce qu'**il est constitué par une poudre contenant au moins 75% de composés minéraux hydrophobes, pressée et imprégnée par un liant liquide volatil, ladite poudre représentant à l'issue de la fabrication entre 80 % et 95 % du poids total et le liant liquide volatil au moins 5 % dudit poids.

12. Produit solide selon la revendication 11, comprenant en poids entre 80 % et 95 % de talc ou de mica et au moins 5 % de liant liquide volatil.

13. Produit solide selon l'une des revendications 11 ou 12, **caractérisé en ce que** le liant liquide volatil est un silicone cyclique, en particulier un cyclométhicone, de viscosité inférieure ou égale à 10 mPa.s (10 centipoises).

14. Produit solide selon l'une des revendications 11,12 ou 13, **caractérisé en ce qu'**il comprend une proportion de liant gras organique, en particulier stéarate métallique, inférieure à la proportion de liant liquide volatil.

15. Produit solide selon la revendication 14, **caractérisé en ce qu'**il comprend sensiblement entre 6 % et 9 % en poids de liant liquide volatil et entre 2 % et 3 % de stéarate métallique.

16. Produit selon l'une des revendications 11, 12, 13, 14 ou 15, contenant, en matières solides, au moins 95 % de talc (poids de talc rapporté au poids de matière solide du produit).

## Patentansprüche

1. Verfahren zur Herstellung eines festen Produktes in handhabbarer Form auf der Basis von hydrophoben Mineralverbindungen, insbesondere für die kosmetische oder hygienische Verwendung, **dadurch gekennzeichnet, dass** ein Pulver, das wenigstens 75% hydrophobe Mineralverbindungen enthält, mit einem flüchtigen Flüssigbindemittel in solchen Gewichtsanteilen imprägniert wird, dass der Anteil an flüchtigem Flüssigbindemittel wenigstens gleich 5% und der Pulveranteil wenigstens gleich 80% ist, und dadurch, dass das erhaltene imprägnierte Pulver in eine Form gepresst wird, so dass ihm eine Kohäsion verliehen wird, die die Starrheit des Produktes gewährleistet.

2. Verfahren nach Anspruch 1, bei dem das Pulver auf Talk oder Glimmer basiert.

3. Verfahren nach Anspruch 1 oder 2, **dadurch gekennzeichnet, dass** als flüchtiges Bindemittel ein zyklisches flüssiges Silikon mit einer Viskosität verwendet wird, die kleiner oder gleich 10 mPa.s ist.

4. Verfahren nach Anspruch 3, **dadurch gekennzeichnet, dass** als flüchtiges Flüssigbindemittel ein zyklisches Silikon der Familie der Cyclomethicone verwendet wird.

5. Verfahren nach Anspruch 1, 2, 3 oder 4, **dadurch gekennzeichnet, dass** das imprägnierte Pulver gepresst wird, indem es einem Pressdruck im Wesentlichen zwischen 200 und 700 kg/cm² in einer zu der gewünschten Gestalt konjugierten Form unterworfen wird, wobei das erhaltene feste Produkt aus der Form genommen und mit Hilfe einer an seine Form angepassten Auflage konditioniert wird, so dass es ergriffen werden kann.

6. Verfahren nach Anspruch 1, 2, 3, 4 oder 5, **dadurch gekennzeichnet, dass** dem Pulver ein Pulver eines organischen Fettbindemittels in einem Gewichtsanteil zugegeben wird, der kleiner ist als der Anteil des flüchtigen Flüssigbindemittels.

7. Verfahren nach Anspruch 6, bei dem dem Pulver ein metallisches Stearat zugegeben wird.

8. Verfahren nach Anspruch 7, **dadurch gekennzeichnet, dass** das imprägnierte Pulver dadurch realisiert wird, dass dem Pulver im Wesentlichen 6 bis 9 Gew.-% an flüchtigem Flüssigbindemittel und 2 bis 3 Gew.-% metallisches Stearatpulver zugegeben wird.

9. Verfahren nach einem der vorherigen Ansprüche, bei dem in das Pulver ein Additiv, insbesondere ein Parfüm oder Weichmacher, in einem Gewichtsanteil kleiner oder gleich 2% zugegeben wird.

10. Verfahren nach einem der vorherigen Ansprüche, bei dem das Pulver wenigstens 95 Gew.-% Talk enthält.

11. Festes Produkt mit einer geeigneten Form und einer Kohäsion, die es handhabbar macht, insbesondere in der Form eines Stiftes, der geeignet ist, bei Kontakt eine mineralische Absonderung zu erzeugen, **dadurch gekennzeichnet, dass** es durch ein Pulver gebildet wird, das wenigstens 75% hydrophobe Mineralverbindungen enthält, mit einem flüchtigen Flüssigbindemittel gepresst und imprägniert, wobei das genannte Pulver am Ende des Herstellungsprozesses zwischen 80 und 95 Gew.-% des Gesamtgewichtes und das flüchtige Flüssigbindemittel wenigstens 5% des genannten Gewichtes repräsentiert.

12. Festes Produkt nach Anspruch 11, das zwischen 80 und 95 Gew.-% Talk oder Glimmer und wenigstens 5% des flüchtigen Flüssigbindemittels umfasst.

13. Festes Produkt nach Anspruch 11 oder 12, **dadurch gekennzeichnet, dass** das flüchtige Flüssigbindemittel ein zyklisches Silikon, insbesondere ein Cyclomethicon, mit einer Viskosität von kleiner oder gleich 10 mPa.s ist.

14. Festes Produkt nach Anspruch 11, 12 oder 13, **dadurch gekennzeichnet, dass** es einen Anteil an organischem Fettbindemittel, insbesondere metallisches Stearat, umfasst, der kleiner ist als der Anteil des flüchtigen Flüssigbindemittels.

15. Festes Produkt nach Anspruch 14, **dadurch gekennzeichnet, dass** es im Wesentlichen 6 bis 9 Gew.-% flüchtiges Flüssigbindemittel und 2 bis 3% metallisches Stearat umfasst.

16. Produkt nach Anspruch 11, 12, 13, 14 oder 15, das in Festmasse wenigstens 95% Talk (Talkgewicht bezogen auf das Gewicht der Festmasse des Produktes) enthält.

## Claims

1. A method of making a solid product in a form capable of being handled based on hydrophobic mineral compounds, in particular for cosmetic or hygienic use, charecterized in that a powder containing at least 75% hydrophobic mineral compounds is impregnated using a volatile liquid binder in proportions by weight such that the proportion of volatile liquid binder is at least equal to 5% and the proportion of powder at least equal to 80%, and in that the resulting impregnated powder is pressed so as to shape it and to provide it with cohesion ensuring the rigidity of the product.

2. A method as claimed in claim 1, wherein the powder is based on talcum or mica.

3. A method as claimed in one of claims 1 or 2, charecterized in that a cyclic liquid silicone with a viscosity less than or equal to 10 mPa.s is used as the volatile binder.

4. A method as claimed in claim 3, charecterized in that a cyclic silicone from the family of the cyclomethicones is used as the volatile liquid binder.

5. A method as claimed in one of claims 1, 2, 3 or 4, charecterized in that the impregnated powder is pressed by subjecting it to a pressing pressure approximately between 200 and 700 kg/cm² in a mold the shape of which is adapted to the desired shape, the resulting solid product being extracted from the mold and packaged using a support adapted to its shape, allowing it to be grasped.

6. A method as claimed in one of claims 1, 2, 3 4 or 5, charecterized in that a powder of an organic fatty binder is added to the powder in a proportion by weight lower than the proportion of volatile liquid binder.

7. A method as claimed in claim 6, wherein a metal stearate is added to the powder.

8. A method as claimed in claim 7, charecterized in that the impregnated powder is made by adding to the powder approximately between 6 wt.% and 9 wt.% of volatile liquid binder and between 2 wt.% and 3 wt.% of metal stearate powder.

9. A method as claimed in one of the above claims, wherein an additive, particularly perfume or emollient, is incorporated in the powder in a proportion by weight of less than or equal to 2%.

10. A method as claimed in one of the above claims, wherein the powder contains at least 95 wt.% talcum.

11. A solid product having a characteristic shape and cohesion making it capable of being handled, particularly in the form of a stick, suitable for creating a mineral deposit by contact, consisting of a powder containing at least 75% hydrophobic mineral compounds, pressed and impregnated with a volatile liquid binder, the said powder representing, at the end of manufacturing, between 80% and 95% of the total weight and the volatile liquid binder at least 5% of the said weight.

12. A solid product as claimed in claim 11, comprising between 80 wt.% and 95 wt.% talcum or mica and at least 5 wt.% volatile liquid binder.

13. A solid product as claimed in one of claims 11 or 12, charecterized in that the volatile liquid binder is a cyclic silicone, in particular a cyclomethicone, with a viscosity less than or equal to 10 mPa.s.

14. A solid product as claimed in one of claims 11, 12 or 13, comprising a proportion of organic fatty binder, in particular metal stearate, which is less than the proportion of volatile liquid binder.

15. A solid product as claimed in claim 14, comprising approximately between 6 wt.% and 9 wt.% volatile liquid binder and between 2 wt.% and 3 wt.% metal stearate.

16. A product as claimed in one of claims 11, 12, 13, 14 or 15, containing, as solids, at least 95% talcum (weight of talcum in relation to the weight of solids in the product).
